# EUROPEAN PATENT SPECIFICATION

(11) **EP 1 594 513 B1**
(45) Date of publication and mention of the grant of the patent: **10.08.2011**
(21) Application number: 04701730.6
(22) Date of filing: 13.01.2004
(51) Int. Cl.: A61K 45/06, A61K 31/485, A61K 47/48

(54) **CARBOHYDRATE CONJUGATES TO PREVENT ABUSE OF CONTROLLED SUBSTANCES**
KOHLENHYDRATKONJUGATE ZUR VERHINDERUNG DES MISSBRAUCHS KONTROLLIERTER SUBSTANZEN
CONJUGUES D'HYDRATES DE CARBONE UTILES POUR EMPECHER L'INTOXICATION AUX MEDICAMENTS REGLEMENTES

(30) Priority: 13.01.2003 US 439468 P
(43) Date of publication of application: 16.11.2005
(73) Proprietor: Shire LLC, Florence, KY 41042 (US)
(72) Inventor: MICKLE, Travis, US CORALVILLE IA 52241 (US); PICCARIELLO, Thomas, Blacksburg, VA 24060-2534 (US); MONCRIEF, James, Scott, Christiansburg, VA 24073 (US); BOERTH, Nancy, Johnston, Blacksburg, VA 24060 (US); BISHOP, Barney, Blacksburg, VA 24060 (US)
(74) Representative: Atkinson, Jonathan David Mark
(86) International application number: PCT/US2004/000646
(87) International publication number: WO 2004/062614

(56) References cited:
- WO-A-02/24715
- WO-A-02/094173
- WO-A-03/057716
- WO-A-03/072046
- US-A1- 2003 130 205
- US-A1- 2004 161 382
- US-B2- 6 713 452

## Description

### CROSS-REFERENCED AND RELATED APPLICATION

This application claims the benefit of U.S. Provisional application 60/439,468 filed January 13, 2003 and is hereby incorporated by reference in its entirety.

### BACKGROUND OF THE INVENTION

### (i) Field of the Invention

The invention relates to novel pharmaceutical compounds and more particularly to controlled substances that are covalently bound to a chemical moiety and thus rendered pharmaceutically inactive until broken down by enzymatic and/or chemical means in a time-dependent manner following oral administration. Delayed release from the conjugate prevents spiking of drug levels and affords gradual release over an extended period of time. The enzymatic and/or chemical conditions necessary for the release of the controlled substance are either not present or of minimal activity when the novel pharmaceutical compound is introduced nasally, inhaled, or injected; thus, also preventing spiking when administered by these routes. Controlled substances with these novel properties are less likely to be abused due to the diminished "rush" effect of the modified controlled substance. Consequently, the therapeutic value of these pharmaceuticals is enhanced by decreasing euphoria while increasing the duration of the analgesic effect.

### (ii) Description of the Related Art

A number of pharmacologically useful compounds are also commonly abused controlled substances. In particular, analgesics that are prescribed for the management of acute and chronic pain have become increasingly abused over the last decades. For example, the increase in prescription of oxycodone in the last few years led to widespread abuse of this drug in certain areas of the U.S. Amphetamines are another example of controlled substances with important pharmacological uses that also are highly addictive and commonly abused. There has been considerable effort in research to develop new compounds with the pharmacological benefits of these drugs, but that are less addictive or less likely to be abused.

The need for "street safe" narcotics was highlighted recently by the epidemic of problems associated with the long-acting analgesic OxyContin, an extended release form of oxycodone. Numerous media reports described the rapidly growing frequency of abuse of this potent narcotic which contains high levels of oxycodone formulated into an extended release matrix. The problem was summarized recently in the following extract from a web page of the National Institute for Drug Abuse (NIDA):
A variety of sources, including NIDA's own Community Epidemiology Work Group, a network of epidemiologists and researchers from 21 major U.S. metropolitan areas who monitor and report on community-level trends in drug abuse, are finding that people are "short circuiting" the time-release form of this medication by chewing, crushing, or dissolving the pills. Chewing or crushing the prescription drug corrupts or foils its time-release protection, enabling the users to experience a rapid and intense euphoria that does not occur when taken as designed and prescribed. Once having crushed the pills, the individuals are injecting, inhaling, or taking them orally, often with other pills, marijuana, or alcohol.

Although the injecting and "snorting" routes of administration are most associated with drug abuse "a Drug Enforcement Administration (DEA) study found that the vast majority of 110 people identified in the previous two years as having overdosed on OxyContin took the drug orally as opposed to snorting or injecting a crushed tablet (see *ADAW,* Nov. 19, 2001). Rapid release of the medication in people who are not tolerant can be fatal."

WO 02/094173 discloses a dosage from including substances adhered to the therapeutic agent which are soluble in the gastrointestinal tract. The dosage froms of this patent are either encapsulated with or modified by acid labide substances or linkages that are particularly susceptible to gastric enzymes.

### SUMMARY OF THE INVENTION

The invention provides a "street-safe" version of a controlled substance which permits the therapeutically beneficial effects of the substance while reducing or eliminating the euphoric effects that lead to substance abuse.

According to one aspect of the present invention, there is provided a pharmaceutical composition comprising: a controlled substance; and a carbohydrate covalently bound to said controlled substance in a manner that renders said controlled substance pharmacologically inactive or substantially diminishes its activity, wherein the controlled substance is selected from the group comprising: codeine, fentanyl, hydrocodone, hydromorphone, Levorphanol, methadone, morphine, oxycodone, propoxyphene, sufentanyl, amphetamine and methylphenidate and, wherein the carbohydrate comprises less than 10 individual carbohydrates.

According to a second aspect of the present invention, there is provided use of the composition as described above in the preparation of an oral medicament for delivering a controlled substance to a patient so as to obtain a therapeutic, but not a substantial euphoric effect.

According to a third aspect of the present invention, there is provided use of the composition of any preceding claim in the preparation of a parenterally administered medicament for delivering a controlled substance to a patient so as to obtain a therapeutic, but not a substantial euphoric effect.

According to a fourth aspect of the present invention, there is provided use of the composition of any preceding claim in the preparation of a medicament for preventing the euphoric effect of a controlled substance comprising providing a controlled substance covalently bound to a carbohydrate.

The invention provides a controlled substance that has been chemically modified to release the controlled substance only under selected conditions which do not give rise to or reduce the euphoric effect. A further feature of the invention permits the release to occur at a controlled rate that does not give rise to or reduces the euphoric effect.

Another embodiment provides a chemically modified controlled release substance that is inactive and resistant to absorption until broken down by chemical or enzymatic means at the desired target location, such as under the acidic conditions of the stomach and/or the enzymatic activity present in the gastrointestinal tract. In a preferred embodiment, the breakdown does not occur until the conjugate has passed into the colon.

One embodiment of the invention provides a composition which resistant to oral abuse through the covalent modification of the substance until it is available for absorption.

In another embodiment of the invention, the chemically modified controlled release substance is released in the colon and or bloodstream, at controlled rate that reduces or does not give rise to a euphoric effect.

In one embodiment, the invention comprises a controlled substance that has been rendered inactive or substantially inactive wherein said controlled substance is covalently bonded to the chemical moiety. In a preferred embodiment the chemical moiety is a carbohydrate, more preferably a carbohydrate chain. The carbohydrate chain preferably comprises between 2 and 50 carbohydrate groups, more preferably the carbohydrate chain is between 2 and 10 carbohydrate groups. Most preferably the carbohydrate is between 2 and 5 carbohydrate groups. In another embodiment the carbohydrate is attached to a peptide and the controlled substance is attached to either the carbohydrate or the peptide.

In another embodiment, the invention comprises a controlled substance that has been rendered inactive or substantially inactive wherein the controlled substance is covalently bonded to a carbohydrate which breaks down under the conditions of the colon thereby providing protection for the active agent (substance) through the stomach.

In an oral composition of the invention, absorption of the controlled substance into the bloodstream occurs in a sustained release manner wherein peak concentrations of the drug are decreased when compared to non-conjugated drug given in a similar dosage and formulation.

Another aspect of the invention relates to a method for delivering a controlled substance to a patient so as to obtain a therapeutic, but not a substantial euphoric effect, comprising orally administering a composition of the invention to a patient.

The invention provides a method for delivering a controlled substance to a patient to obtain a therapeutic effect without a substantial euphoric effect, comprising parenterally administering the above composition to the patient.

### BRIEF DESCRIPTION OF THE DRAWINGS

Fig. 1. illustrates mean hydrocodone serum levels compared to a ribose-hydrocodone conjugate orally delivered.
Fig. 2. illustrates mean hydrocodone serum levels compared to a ribose-hydrocodone conjugate intranasally delivered.
Fig. 3. illustrates mean hydrocodone serum levels compared to a ribose-hydrocodone conjugate intravenously delivered.
Fig. 4. illustrates mean hydrocodone serum levels compared to a galactose-hydrocodone conjugate orally delivered.
Fig. 5. illustrates mean hydrocodone serum levels compared to a galactose-hydrocodone conjugate intranasally delivered.

### DETAILED DESCRIPTION OF PREFERRED EMBODIMENTS

The invention provides methods for altering controlled substances in a manner that decreases their potential for abuse. The novel compositions may be combined in tablets with suitable excipients or formulated in solution for oral delivery. When delivered by the oral route the controlled substance is released in a time-dependent manner (sustained release) by acid hydrolysis and/or enzymatic cleavage. When administered by injection the controlled substance is released in a time-dependent manner (sustained release) by way of serum enzymes.

### Terms defined

Controlled substance - a substance subject to federal regulation of its manufacture, sale, or distribution because of the potential for, or evidence of, abuse; its potential for psychic or physiological dependence; it constitutes a public health risk; scientific evidence of its pharmacologic effect; or its role as a precursor of other controlled substances.

Chemical moiety - a substance made up of chemical elements and characterized by a defined molecular composition. It can exist as a part of the drug conjugate and can be separated from the conjugate. Examples include a carbohydrate or chain of carbohydrates, an amino acid, an oligopeptide, or a polypeptide, but may be any number of other substances.

Although the discussion which follows focuses on oral administration of the controlled substance, it will be appreciated that the compositions and methods of the invention are likewise applicable to other forms of administration, for example, injectable administration of the controlled substance.

Covalent attachment of a chemical moiety to a controlled substance renders the substance pharmacologically inactive and resistant to absorption. Removal of the chemical moiety by enzymatic or chemical means, however, restores the activity and the ability to be absorbed. The conditions of the colon, stomach and/or the enzymatic activity present in the targeted portion of the gastrointestinal tract can therefore affect release of the active controlled substance.

When abused, controlled substances are typically delivered by means other than the oral route, namely by: i) parenteral injection; ii) intranasal delivery; or iii) inhalation. Administration by these routes results in rapid absorption into the bloodstream and the subsequent "rush" effect sought by the user or addict. By contrast, when given by these routes, the covalently modified compound of the invention (adopted for breakdown in the stomach or intestinal tract) is: i) not exposed to the necessary chemical and/or enzymatic conditions for release of the active agent; or ii) the required activity is not present in sufficient amounts to affect rapid release/absorption. The covalently modified controlled substance, therefore, does not produce the euphoric effect sought by users or addicts, but remains effective as a therapeutic.

The invention may be comprised of any controlled substance covalently attached to any chemical moiety, such as narcotics. Preferably, the controlled substance is an analgesic or stimulant. Further, the controlled substance is preferably selected from the following analgesics: codeine, fentanyl, hydrocodone, hydromorphone, levorphanol, methadone, morphine, oxycodone, propoxyphene, and sufentanyl. The controlled substance may also be amphetamine or methylphenidate.

The chemical moiety comprising the invention may be any chemical substance that can be attached to the controlled substance in a manner that renders it pharmacologically inactive. Analgesics and stimulants produce their pharmacological effects through binding to specific receptors or uptake proteins. The attachment of certain chemical moieties can therefore prevent the active substance from binding to receptor(s) or a recognition site on an uptake protein. Further, without wishing to be bound to the theory, the covalent modification is believed to prevent the pharmacological effect by preventing the drug from crossing the blood-brain barrier. Preferably, the attachment of the chemical moiety to the controlled substance will also prevent or substantially delay the absorption of the compound, particularly when the compound is delivered by routes other than oral administration.

Preferably, the attached chemical moiety is a carbohydrate. The carbohydrate chain preferably comprises less than 100 groups, more preferably less than 50 groups and still more preferably less than 10 groups. In another embodiment the carbohydrate comprises less than 5 individual carbohydrates. In another embodiment the carbohydrate comprises 4 individual carbohydrates. In another embodiment the carbohydrate comprises 3 individual carbohydrates. In another embodiment the carbohydrate comprises 2 individual carbohydrates. In another embodiment the carbohydrate the carbohydrate comprises a single carbohydrate.

The carbohydrate is also preferably a sugar. In a preferred embodiment the sugar is selected from ribofuranose, ribose, galactose, galactopyranose, xylose, mannofuranose, or combinations thereof.

The attached chemical moiety may be comprised of other naturally occurring or synthetic substances. Controlled substances, for example, could also be attached to lipids, amino acids, polypeptide, nucleic acids, or vitamins. These chemical moieties could be expected to serve the same functions as a carbohydrate; namely, affect delayed release in the gastrointestinal tract and prevent rapid absorption of the active agent.

In one embodiment, the covalently attached chemical moiety is removed by enzymatic activity encountered by the compound in the stomach and/or intestinal tract. The stomach and intestinal tract are bathed in degradative enzymes. For example, the pancreas releases into the small intestine a myriad of hydrolytic enzymes such as glycosidases, proteases, lipases, and amylases, and nucleases. Additionally, the intestinal epithelial cells that line the surface of the GI tract produce various surface associated and intracellular degradative enzymes (e.g. brush border peptidases, esterases). These enzymes degrade proteins, lipids, carbohydrates, and nucleic acids contained in ingested food. Thus, it can be expected that the controlled substance will be released from the attached chemical moiety when the appropriate enzyme(s) is encountered in the gastrointestinal tract.

In another embodiment, the chemical moiety is attached to the controlled substance in a manner in which it is not readily released by conditions found in the mouth (saliva), the intranasal cavity, the surface of the lungs, or in the serum. Extreme acid conditions encountered in the stomach are not present elsewhere in humans. Therefore, any acid dependent release mechanism will occur only after oral administration. Although, degradative enzymes are present is the aforementioned environments, they are not generally present in the high concentrations found in the intestinal tract. Thus, release of the controlled substance by enzymatic cleavage will not occur rapidly when the novel compounds are administered by routes other than oral delivery.

In a specific embodiment of the invention, the analgesic (*e.g.* oxycodone or hydrocodone) is attached to a ribofuranose (or other combinations of sugars such as ribose or furanose). The resulting ester linkages can be hydrolyzed by glycosidases encountered in the gastrointestinal tract. Glycosidases are not present at high levels in saliva or on the mucosal surfaces of the nasal cavity, lungs, or oral cavity. Thus, controlled substances attached to ribofuranose by this method would not be rapidly released by saliva or when delivered intranasally or by inhalation.

For each of the recited embodiments, compositions and methods of using the invention may further be formulated or dosed according to industry standards. Further information is also described for instance in Remington's pharmaceutical sciences (18th ed.)(Gennaro, A.R. (Ed.). Easton, PA: Mack Pub. Co. (1990)); Physicians' desk reference (PDR). Montvale, NJ: Medical Economics Co., Annual; and USP DI (United States Pharmacopeia Dispensing Information). Rockville, Md.: United States Pharmacopeial Convention, Annual with supplements. Vol. IA & B: "Information for the health care provider", Vol. II: "Advice for the patient", Vol. III: "Approved drug products and legal requirements". Additionally, to the extent that the solubility or formulation characteristics of the compositions vary from the active agent alone, one skilled in the art may use recognized procedures to properly formulate and determine the appropriate dose for the compositions.

The following examples are given by way of illustration and in no way should be construed as limiting as to the full scope of the invention. Other embodiments and features of the invention will be obvious from the figures and tables.

### EXAMPLES

Hydrocodone, an opioid agonist, was chosen as a model compound for testing conjugates for the hypothesis that conjugates of opioid drugs can afford extended release, while also lowering the potential for abuse.

### Example 1: Preparation of the Chloroformate of 2,3-O-isopropylidene-1-methoxy-D-ribofuranose

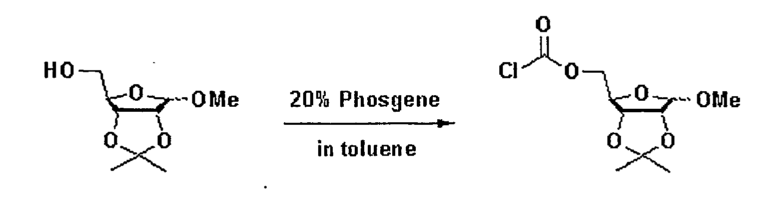

| Reagents | MW | Weight | mmoles | Molar Equivalents |
|---|---|---|---|---|
| 2,3-*O*-isopropylidene-1-methoxy-D-ribofuranose | 204 | 1.00g | 3.85 | 1 |
| 20% Phosgene in toluene | - | 25ml | - | - |

### Chloroformate of 2,3-O-isopropylidene-1-methoxy-D-ribofuranose

To a stirring solution of 20% phosgene in toluene under an inert atmosphere was added 2,3-*O*-isopropylidene-1-methoxy-D-ribofuanose via syringe. The resulting clear, colorless solution was stirred at ambient temperature for 30 minutes. After stirring, Ar(g) was bubbled through the solution for approximately 20 minutes to remove any excess phosgene. Solvent was then removed and product dried under vacuum for 18 hours. Product was used without further purification or characterization.

### Example 2: Preparation of Ribo-Hydrocodone

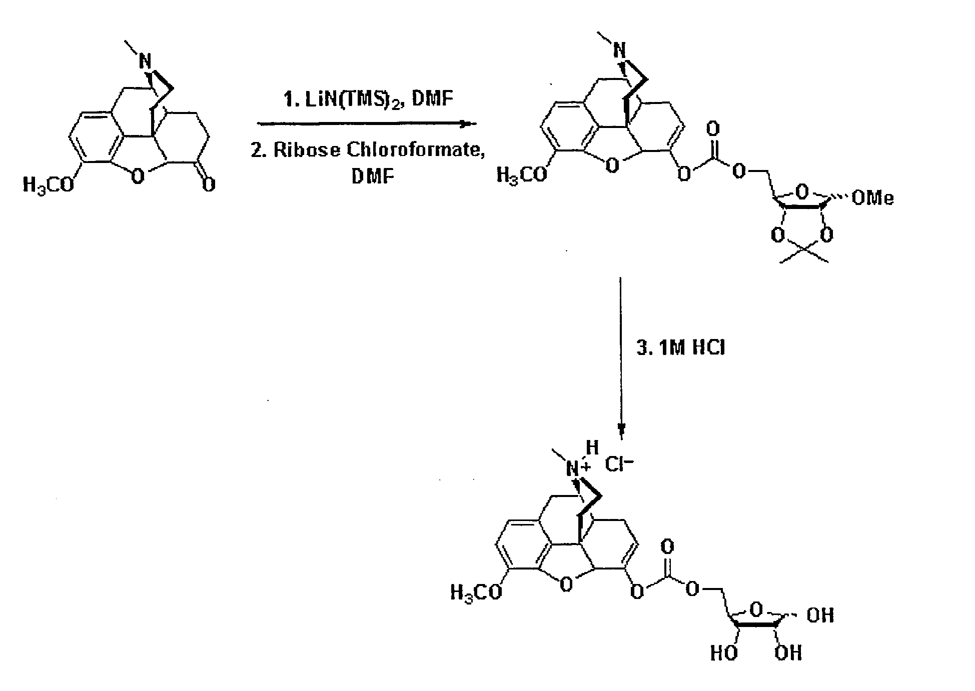

| Reagents | MW | Weight | mmoles | Molar Equivalents |
|---|---|---|---|---|
| 1. Hydrocodone | 299 | 0.733g | 2.45 | 1.0 |
| 1. LiN(TMS)₂ in THF | 1M | 3.68ml | 3.68 | 1.5 |
| 1. DMF | - | 8ml | - | - |
| 2. Ribose Chloroformate | - | - | 4.90 | 2.0 |
| 2. DMF | - | 3ml | - | - |
| 3. 1M HCl | 1M | 10ml | - | - |

### Ribo-Hydrocodone:

To a solution of hydrocodone in DMF was added LiN(TMS)₂ in THF via syringe. The solution was stirred at ambient temperatures for 5 minutes then the chloroformate of ribose in DMF was added via syringe. The resulting solution was stirred at ambient temperatures for 2 hours. A TLC was taken (9:1 CHCl₃:MeOH; UV and 5% H₂SO₄ in MeOH; R_{f(product)} = ∼0.5). Reaction was neutralized to pH 7 with 1M HCl. Solvent was removed. Crude product was taken up in CHCl₃ (50ml), washed with water (3 X 50ml), dried over MgSO₄, filtered and solvent removed. Final product was purified using preparative HPLC (10mM CH₃COONH₄ / MeCN; 0-20min: 80/20 → 0/100). Solid was collected as a clear, colorless glass (0.095g, 7% yield): ¹H NMR (DMSO-d₆) δ 1.26 (s, 3H), 1.39 (s, 3H), 1.50 (m, 2H), 1.89 (s, 4H), 2.08 (m, 2H), 2.29 (s, 4H), 2.40 (m, 2H), 2.88 (d, 1H), 3.08 (m, 1H), 3.25 (s, 3H), 3.73 (s, 3H), 4.12 (m, 2H), 4.28 (t, 1H), 4.58 (d, 1H), 4.72 (d, 1H), 4.97 (s, 1H), 4.98 (s, 1H), 5.70 (s, 1H), 6.66 (d, 1H), 6.75 (d, 1H). MS Calculated mass = 529.2 Found = 530.4 (M+H).

To the protected ribose intermediate was added 10ml of 1M HCl. The resulting solution was stirred at ambient temperatures for 2 hours. Solvent was removed and final product dried under vacuum. Solid was collected as a waxy, slightly yellow solid (0.092g, quant.): ¹H NMR (DMSO-d₆) δ 1.51 (t, 1H), 1.83 (d, 1H), 2.41 (dt, 1H), 2.27 (t, 1H), 2.63 (dd, 1H), 2.80 (s, 3H), 2.96 (m, 2H), 3.20 (m, 1H), 3.75 (s, 3H), 3.82-4.34 (br m, 12H), 5.15 (s, 1H), 5.72 (s, 1H), 6.75 (d, 1H), 6.88 (d, 1H), 11.37 (br s, 1H).

### Example 3: Preparation of Galacto-Hydrocodone

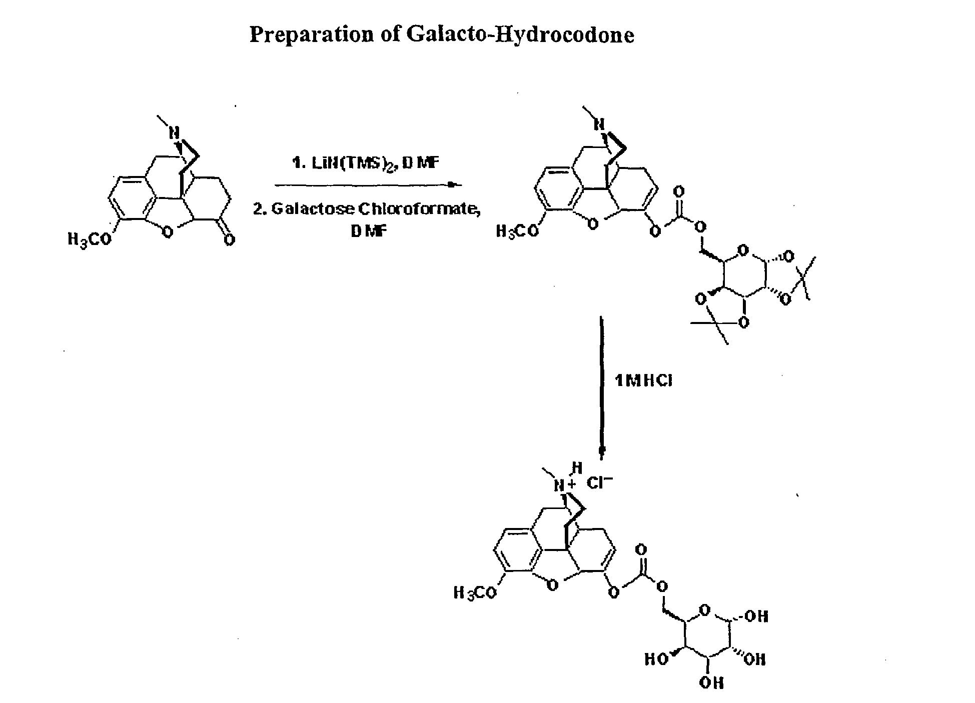

| **Reagents** | **MW** | **Weight** | **mmoles** | **Molar Equivalents** |
|---|---|---|---|---|
| **1. Hydrocodone** | **299** | **0.223g** | **0.75** | **1.0** |
| **1. LiN(TMS)₂ in THF** | **1M** | **1.13ml** | **1.13** | **1.5** |
| **1. DMF** | **-** | **5ml** | **-** | **-** |
| **2. Galactose Chloroformate** | **-** | **-** | **1.49** | **2.0** |
| **2. DMF** | **-** | **3ml** | **-** | **-** |
| **3. 1M HCl** | **1M** | **30ml** | **-** | **-** |
| **3. Acetone** | **-** | **20ml** | **-** | **-** |

### Galacto-Hydrocodone

To a solution of hydrocodone in DMF was added LiN(TMS)₂ in THF via syringe. The solution was stirred at ambient temperatures for 5 minutes then the chloroformate of galactose in DMF was added via syringe. The resulting solution was stirred at ambient temperatures for 2 hours. A TLC was taken (9:1 CHCl₃:MeOH; UV and 5% H₂SO₄ in MeOH; R_{f(product)} = ∼0.5). Reaction was neutralized to pH 7 with 6M HCl. Solvent was removed. Final product was purified using preparative TLC (0-10% MeOH in CHCl₃). Solid was collected as a white powder (0.180g, 41% yield): ¹H NMR (DMSO-d₆) δ 1.28 (2s, 6H), 1.37 (s, 3H), 1.44 (3, 3H), 1.49 (m, 2H), 1.88 (dt, 1H), 2.08 (m, 2H), 2.29 (s, 4H), 2.40 (m, 2H), 2.90 (d, 1H), 3.09 (s, 1H), 3.73 (s, 3H), 3.99 (dd, 1H), 4.14 (t, 1H), 4.26 (dt, 2H), 4.39 (d, 1H), 4.63 (d, 1H), 4.95 (s, 1H), 5.48 (d, 1H), 5.68 (d, 1H), 6.65 (d,1H), 6.74 (d, 1H); MS Calculated mass = 585.6 Found = 586.4 (M+H).

To the protected galactose intermediate was added 30ml of 1M HCl and 20ml acetone. The resulting solution was stirred at ambient temperatures for 3 hours. Solvent was removed and final product dried under vacuum. Solid was collected as a white solid: MS Calculated mass = 505.5 Found = 506.4 (M+H).

### Example 4. Preparation of the Chloroformate of 1,2:3,4-di-O-isopropylidene-D-galactopyranose

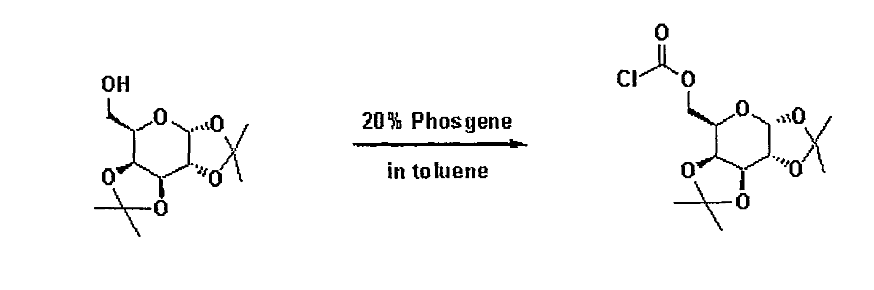

| **Reagents** | **MW** | **Weight** | **mmoles** | **Molar Equivalents** |
|---|---|---|---|---|
| **1,2:3,4-di-*O*-isopropylidene-D-galactopyranose** | **260** | **1.00g** | **3.85** | **1** |
| **20% Phosgene in toluene** | **-** | **20ml** | **-** | **-** |

### Chloroformate of 1,2:3,4-di-O-isopropylidene-D-galactopyranose

To a stirring solution of 20% phosgene in toluene under an inert atmosphere was added 1,2:3,4-di-*O*-isopropylidene-D-galactopyranose via syringe. The resulting clear, colorless solution was stirred at ambient temperature for 30 minutes. After stirring, Ar(g) was bubbled through the solution for approximately 20 minutes to remove any excess phosgene. Solvent was then removed and product dried under vacuum for 18 hours. Product was used without further purification or characterization.

### Example 5: Preparation of Disaccharide-Hydrocodone

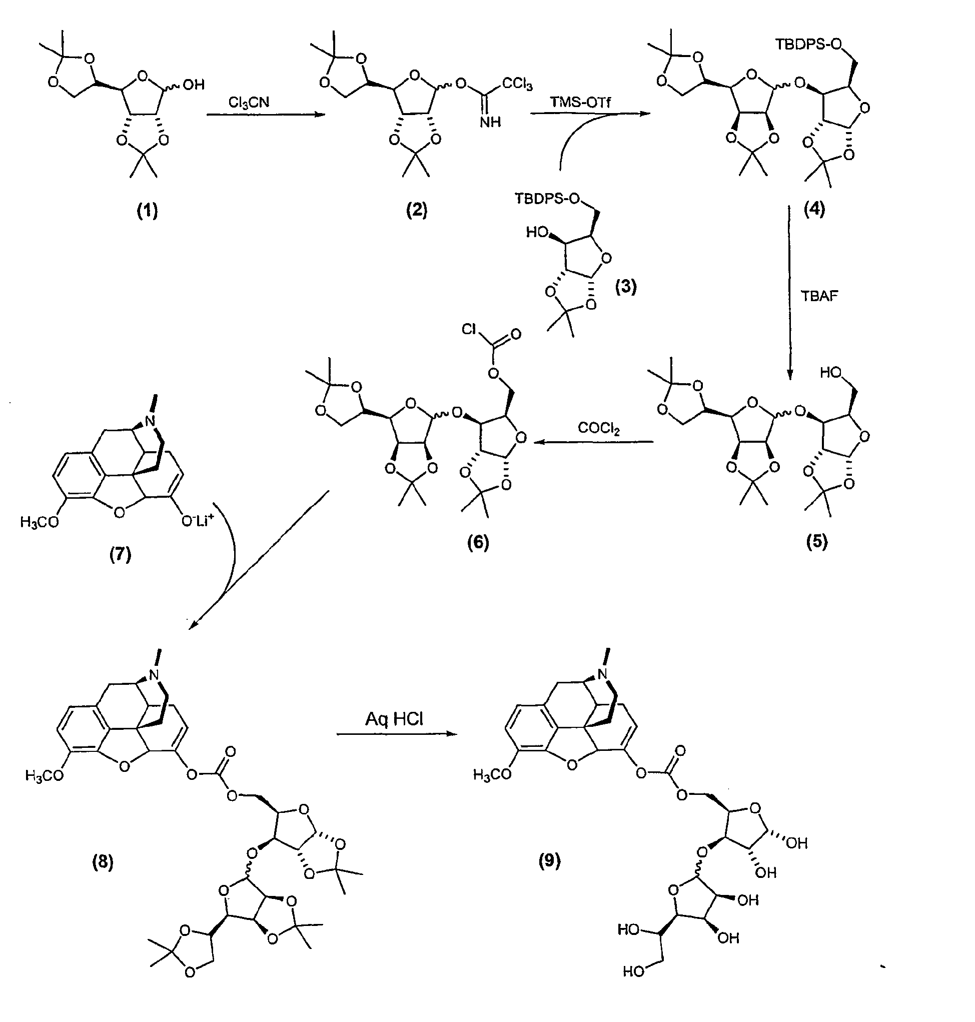

### General scheme for preparation of hydrocodone-disaccharide conjugates:

The protected mannofuranose **(1)** has been converted to the trichloroacetimidate **(2)** as described below. Based on literature precedent, this can in turn be coupled to an orthogonally-protected xylose **(3),** which affords the corresponding disaccharide **(4).** Disaccharide formation is promoted by the addition of a catalytic amount of acid.

Use of an orthogonal protection scheme allows the selective removal of the silyl protecting group using tetrabutyl ammonium fluoride in the presence of the isopropylidene groups, affording the free primary alcohol **(5).** Employing methods already described in the preparation of galactose and ribose conjugates, this alcohol can then be converted to the chloroformate **(6)** and in turn coupled to the hydrocodone-enolate **(7),** resulting in the carbonate **(8).** Deprotection of **(8)** using standard protocols affords the hydrocodone-disaccharide conjugate **(9)**

### Preparation of the trichloroacetimidate of mannofuranose (2):

Dissolved 2,3:5,6-Di-O-isopropylidene-D-mannofuranose (**1**, 0.50g, 1.9mmol) in 5ml of anhydrous dichloromethane. Then, trichloroacetonitrile (0.67ml, 6.7mmol) was added to the solution followed by dry K₂CO₃ (0.54g, 0 3.8 mmol). The reaction was then allowed to stir over night at room temperature under argon. Qualitative thin-layer chromatography (2:1 hexanes/acetone) of the reaction mixture indicated that the desired trichloroacetimidate had been formed, based on the disappearance of the spot corresponding to the mannofuranose starting material which correlated with the appearance of a new faster-running spot. This is consistent with literature precedence. The reaction was then filtered through fritted glass and the filtrate collected and freed of solvent by rotary-evaporation under high vacuum. This resulted in a viscous oil that solidified with storage over night under high vacuum.

### Example 6: Stability of Narcotics in the "Kitchen Test"

To determine the accessibility of active narcotic from the synthetic conjugates, an attempt was made to release the narcotic from its conjugate. In a kitchen test, we determined the stability of the conjugates when subjected to conditions that would be available to any drug addict. Thus, the kitchen test is a model of "street-safeness" for the narcotics conjugates. The conjugates were heated in a water bath (80-90°C) in a pH range (1-12) over a 1 hour time course. This pH range constitutes about any solution that can be found commercially at any grocery or drug store. The release of the narcotic from the conjugate was monitored by HPLC and quantified with a dose-response calibration curve of the parent narcotic.

Using only solutions that would be available to an addict, conjugates were dissolved in water. Water insoluble conjugates were either (a) dissolved in a minimum amount (≤5% v/v) of organic solvent [e.g., DMSO, methanol, or ethanol], (b) added as a suspension to the assay, or (c) dissolved completely in chloroform, and aliquoted to a test tube where the chloroform is evaporated, leaving only a known amount of conjugate. Upon preparation of the conjugate, the conjugate is added to the various pH solutions and heated for 0, 5, 15, or 60 minutes. At the indicated time point, each test tube was removed from the water bath and neutralized with a chilled phosphate buffer (100mM, pH 7, 4°C).

The kitchen test proceeds as follows:
1. pH solutions [1, 4, 7, 9,12] prepared with HPLC grade H₂O and titrated with NaOH or HCl
2. prepare hot water bath, keeping temperature at 80-90°C
3. conjugates prepared at a concentration of 1mg/ml as indicated above
4. 250µl of conjugate added to 750µl of pH solutions (fmal assay volume is 1ml; if conjugate has been evaporated from CHCl₃, add 1ml of pH solution to test tube)
5. immediately following the addition of conjugate to pH solution, place test tube in hot water bath (80-90°C)
6. at indicated time point, remove test tubes from heat and neutralize with 1ml of chilled phosphate buffer [0 time point is not heated, but immediately neutralized with phosphate buffer after the addition of conjugate to each pH solution]
7. before aliquoting each sample for HPLC analysis, the volume of each tube is adjusted to 2ml to account for changes in concentration from evaporation

Following HPLC analysis, the amount of released narcotic was extrapolated from a calibration curve of the parent drug and plotted as % narcotic released (w/w, based upon theoretical loading of conjugated species) vs. time.

Ribose-Hydrocodone conjugate TM34 was analyzed by the kitchen test. The results of the percent (relative to time zero) increase in free hydrocodone over time is presented in the following table.

**Kitchen Test Stability of Ribose-Hydrocodone**

| Solution | Minutes | | |
|---|---|---|---|
| | 5 | 15 | 60 |
| | Percent Hydrocodone released * | | |
| pH 1 | 8 | 18 | 27.8 |
| pH4 | 11 | 12.6 | 19.6 |
| pH 7 | 12 | 16 | 23.4 |
| pH 9 | 9 | 11 | 21 |
| pH 12 | 44 | 33 | 41.1 |

| | | | |
|---|---|---|---|
| * Relative to zero hour level of free Hydrocodone | | | |

The Ribose conjugate is relatively stable at pHs other than pH 12, with the amount of hydrocodone released being 27.8 % or less after one hour at 90 ° C. At pH 12 less than half the hydrocodone was released from the conjugate and no further release occurred after 5 minutes.

### Example 7: Oral Bioavailablity of Hydrocodone vs. Ribose-Hydrocodone

Doses of Hydrocodone Bitartarate and Ribose-Hydrocodone (TM34) containing equivalent amounts of hydrocodone (0.2143 mg) were administered in gelatin capsules to Male Sprague-Dawley rats (approximately 300 g). The Hydrocodone content of Ribose-Hydrocodone conjugate was determined to be 66% byNMR.

**Table 1. Oral Administration - Ribose-Hydrocodone vs. Hydrocodone-Bitartarate - Serum Levels (ng/ml )**

| Hours | Ribose-HC | Ribose-HC | Ribose-HC | Ribose-HC | Hydrocodone | Hydrocodone | Hydrocodone | Hydrocodone |
|---|---|---|---|---|---|---|---|---|
| | #1 | #2 | #3 | #4 | #1 | #2 | #3 | #4 |
| 0 | 0 | 0 | 0 | 0 | 0 | 0 | 0 | 0 |
| 0.5 | 12 | 7.3 | 12.9 | 12.3 | 10.2 | 14.8 | 6.3 | 8.6 |
| 1.5 | 8.4 | 11 | 8.7 | 10.6 | 6.8 | 7.9 | 10.1 | 4.7 |
| 3 | 2.4 | 7.6 | 2 | 1.6 | 0.6 | 8.3 | 3.5 | 0.2 |
| 5 | 1.3 | 2.6 | 0.2 | 0.5 | 4.6 | 0.7 | 2.7 | 0.1 |
| 8 | 1.1 | 2.9 | 0.8 | 1.2 | 2.1 | 2.1 | 0.9 | 5.5 |
| 12 | 1.2 | 2.2 | 0.7 | 0.9 | 0.5 | 1 | 0.9 | 2.6 |
| **AUC** | **33** | **54** | **29** | **33** | **37** | **47** | **35** | **37** |
| | **Ave. AUC +/- SD** | | **37** | **11** | **Ave. AUC +/- SD** | | **39** | **5** |

**Table 2. Oral Administration - Mean Serum Levels (ng/ml +/- SD)**

| | Ribose-HC | | Hydrocodone | |
|---|---|---|---|---|
| Hours | Ave. | SD | Ave. | SD |
| 0 | 0 | 0 | 0 | 0 |
| 0.5 | 11.1 | 2.6 | 10.0 | 3.6 |
| 1.5 | 9.7 | 1.3 | 7.4 | 2.3 |
| 3 | 3.4 | 2.8 | 3.2 | 3.7 |
| 5 | 1.2 | 1.1 | 2.0 | 2.0 |
| 8 | 1.5 | 0.9 | 2.7 | 2.0 |
| 12 | 1.3 | 0.7 | 1.3 | 0.9 |

**Table 3. Oral Administration - Cmax (ng/ml) of Ribose-Hydrocodone vs. Hydrocodone**

| Sample | Rat #1 | Rat #2 | Rat #3 | Rat #4 | **Ave.** | **SD** |
|---|---|---|---|---|---|---|
| Ribose-HC | 12 | 11 | 12.9 | 12.3 | **12.1** | **0.8** |
| Hydrocodone | 10.2 | 14.8 | 10.1 | 8.6 | **10.9** | **2.7** |

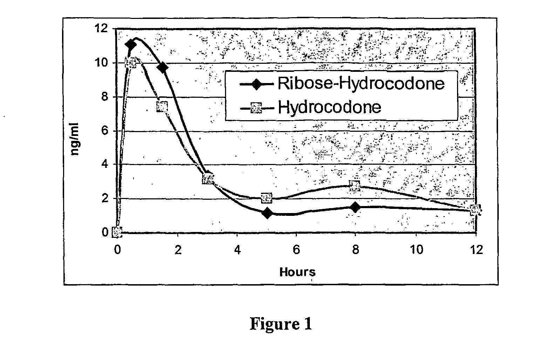

Bioavailability of Ribose-Hydrocodone TM34 was (approximately equal to that of Hydrocodone-Bitaratarate when administered orally (Tables 1-3). The area under the curve (AUC) for Ribose-Hydrocodone was 95% of the AUC for Hydrocodone-Bitartarate (37 vs. 39, respectively). The mean peak serum concentration (Cmax) of Ribose-Hydrocodone was 111% of that of Hydrocodone-Bitratarate (12.1 vs. 10.9, respectively). The serum concentration curves of Ribose-Hydrocodone (TM34) vs. Hydrocodone-Bitartarate administered orally are shown in Fig. 1.

### Example 8: Intranasal Bioavailablity of Hydrocodone vs. Ribose Hydrocodone

Doses of Hydrocodone Bitartarate and Ribose-Hydrocodone (TM34) containing equivalent amounts of Hydrocodone (0.2143 mg) were administered intranasally to Male Sprague-Dawley rats (approximately 300 g). Doses were administered in phosphate buffered saline directly into the nasal flares of the rats.

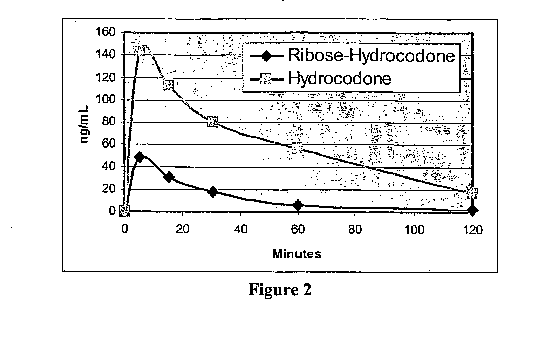

Bioavailability of Ribose-Hydrocodone TM34 was decreased when administered intranasally as compared to that of Hydrocodone-Bitartarate administered by the same route. The AUC of Ribose-Hydrocodone was 20 % of the AUC of Hydrocodone (1,490 vs. 7,303, respectively). Further, C*max* of Ribose-Hydrocodone was 36 % of the AUC of Hydrocodone (51 vs. 143, respectively). The serum concentration curves of Ribose-Hydrocodone (TM34) vs. Hydrocodone-Bitartarate administered intranasally are shown in Fig. 2.

### Example 9: Intravenous Bioavailablity of Hydrocodone vs. Ribose Hydrocodone

Doses of Hydrocodone Bitartarate and Ribose-Hydrocodone (TM34) containing equivalent amounts of Hydrocodone (0.2143 mg) were administered intravenously to Male Sprague-Dawley rats (approximately 300 g). Doses were administered by tail vein injection in phosphate buffered saline.

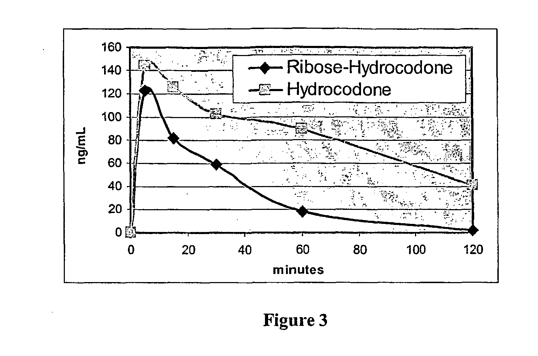

Bioavailability of Ribose-Hydrocodone TM34 was decreased when administered intravenously as compared to that of Hydrocodone-Bitartarate administered by the same route. The AUC of Ribose-Hydrocodone was 41 % of the AUC of Hydrocodone (4,145 vs. 10,233, respectively). C*max* of Ribose-Hydrocodone was 86 % of the AUC of Hydrocodone (123 vs. 143, respectively), thus decreased bioavailability was substantially the result of an increased clearance rate for Ribose-Hydrocodone. The serum concentration curves of Ribose-Hydrocodone (TM34) vs. Hydrocodone-Bitartarate administered intravenously are shown in Fig. 3.

Collectively, examples 7 through 9 illustrate that attachment of a ribofuranose moiety to the C6 position of Hydrocodone affords a compound with decreased potential for abuse. Oral bioavailability of this compound is maintained, whereas intranasal and intravenous bioavailability are substantially decreased thereby diminishing the euphoric effect of the compound when administered by these routes. Further, example 8 illustrates that absorption of the Ribose-Hydrocodone conjugate through the intranasal membrane is substantially blocked indicating that the ability to permeate cell membranes, likely including the blood brain barrier, is diminished. This property may further decrease the potential for abuse by either intranasal or intravenous administration of narcotic conjugates since the narcotic must permeate the blood brain barrier to elicit euphoria. Example 9 illustrates an increased clearance rate for intravenously administered Ribose-Hydrocodone conjugate, providing an additional mechanism for decreased potential abuse of narcotic conjugates.

### Example 10: Oral Bioavailablity of Hydrocodone vs. Galactose-Hydrocodone

Doses of Hydrocodone Bitartarate and Galactose-Hydrocodone (TMb20) containing equivalent amounts of hydrocodone (0.2143 mg) were administered in gelatin capsules to Male Sprague-Dawley rats (approximately 300 g).

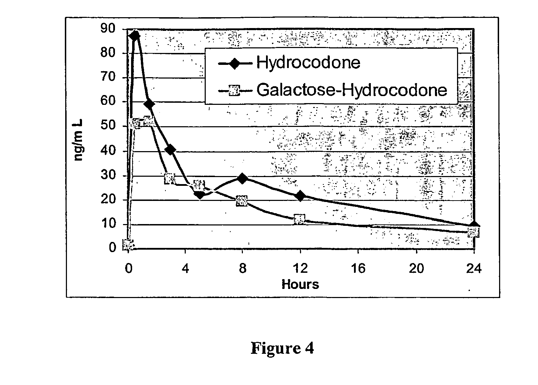

Bioavailability of Galactose-Hydrocodone (TMb20) approached that of Hydrocodone-Bitaratarate when administered orally. The area under the curve (AUC) for Galactose-Hydrocodone was 70% of the AUC for Hydrocodone-Bitartarate (422 vs. 601, respectively). The mean peak serum concentration (C*max*) of Galactose-Hydrocodone was 72% of that of Hydrocodone-Bitratarate (61 vs. 85, respectively). The serum concentration curves of Galactose-Hydrocodone (TMb20) vs. Hydrocodone-Bitartarate administered orally are shown in Fig. 4.

### Example 11: Intranasal Bioavailablity of Hydrocodone vs. Galactose Hydrocodone

Doses of Hydrocodone Bitartarate and Galactose-Hydrocodone (TM34) containing equivalent amounts of Hydrocodone (0.2143 mg) were administered intranasally to Male Sprague-Dawley rats (approximately 300 g). Doses were administered in phosphate buffered saline directly into the nasal flares of the rats.

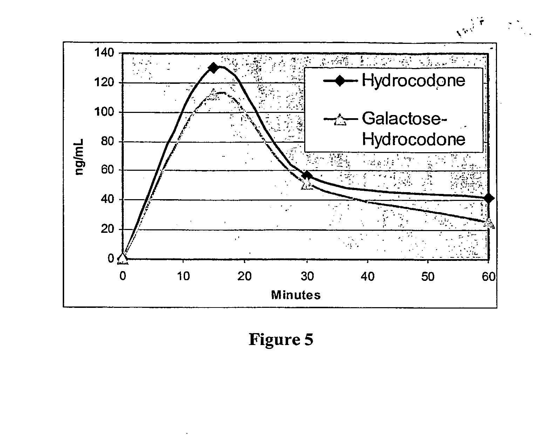

Bioavailability of Galactose-Hydrocodone TMb20 was marginally decreased when administered intranasally as compared to that of Hydrocodone-Bitartarate administered by the same route. The AUC of Galactose-Hydrocodone was 83 % of the AUC of Hydrocodone (3,203 vs. 3,845, respectively). Further, C*max* of Galactose-Hydrocodone was 36 % of the AUC of Hydrocodone (130 vs. 112, respectively). The serum concentration curves of Galactose-Hydrocodone (TM34) vs. Hydrocodone-Bitartarate administered intranasally are shown in Fig. 5.

## Claims

1. A pharmaceutical composition comprising:
a controlled substance; and
a carbohydrate covalently bound to said controlled substance in a manner that renders said controlled substance pharmacologically inactive or substantially diminishes its activity,
wherein the controlled substance is selected from the group comprising: codeine, fentanyl, hydrocodone, hydromorphone, levorphanol, methadone, morphine, oxycodone, propoxyphene, sufentanyl, amphetamine and methylphenidate and,
wherein the carbohydrate comprises less than 10 individual carbohydrates.

2. The composition as claimed in claim 1, wherein said carbohydrate is a sugar.

3. The composition as claimed in claim 2, wherein said sugar is selected from ribofuranose, ribose, galactose, galactopyranose, xylose, mannofuranose, or combinations thereof

4. The composition as claimed in any preceding claim, wherein the carbohydrate comprises less than 5 individual carbohydrates.

5. The composition as claimed in any preceding claim, wherein the carbohydrate comprises 4 individual carbohydrates.

6. The composition as claimed in any preceding claim, wherein the carbohydrate comprises 3 individual carbohydrates.

7. The composition as claimed in any preceding claim, wherein the carbohydrate comprises 2 individual carbohydrates.

8. The composition as claimed in any preceding claim, wherein the carbohydrate comprises a single carbohydrate.

9. The composition as claimed in any preceding claim, wherein said carbohydrate is ribofuranose.

10. The composition as claimed in any preceding claim, wherein said carbohydrate is bound to said controlled substance by an ester or carbonate bond.

11. The composition as claimed in any preceding claim, wherein said controlled substance is codeine.

12. The composition as claimed in claims 1-10, wherein said analgesic is fentanyl.

13. The composition as claimed in claims 1-10, wherein said analgesic is hydrocodone.

14. The composition as claimed in claims 1-10, wherein said analgesic is hydromorphone.

15. The composition as claimed in claims 1-10, wherein said analgesic is levorphanol.

16. The composition as claimed in claims 1-10, wherein said analgesic is methadone.

17. The composition as claimed in claims 1-10, wherein said analgesic is morphine.

18. The composition as claimed in claims 1-10, wherein said analgesic is oxycodone.

19. The composition as claimed in claims 1-10, wherein said analgesic is propoxyphene.

20. The composition as claimed in claims 1-10, wherein said analgesic is sufentanyl.

21. The composition of claims 1-10, wherein said controlled substance is amphetamine.

22. The composition of claims 1-10, wherein said controlled substance is methylphenidate.

23. The composition of any preceding claim, that is resistant to release of said controlled substance in a pharmacologically active form when administered by parenteral injection thus diminishing or preventing a euphoric effect.

24. The composition of claims 1-22, that releases said controlled substance in a pharmacologically active form when administered to a human patient by the oral route.

25. The composition of claims 1-22, that is resistant to release and/or absorption of said controlled substance in a pharmacologically active form when administered by the intranasal route thus diminishing or preventing a euphoric effect.

26. The composition of claims 1-22, that is resistant to release and/or absorption of said controlled substance in a pharmacologically active form when administered by inhalation thus diminishing or preventing a euphoric effect.

27. The composition of claims 1-22, wherein said controlled substance is released in active form in the presence of acid in the stomach or in the presence of enzymes in the intestinal tract or blood serum.

28. The composition of claims 1-22, formulated for oral administration, wherein said controlled substance is released in active form in the presence of acid present in the stomach or in the presence of enzymes present in the intestinal tract.

29. The composition of claims 1-22, that is resistant to absorption across the blood brain barrier of said controlled substance in a pharmacologically active form when administered by parenteral injection thus diminishing or preventing a euphoric effect.

30. The composition of any preceding claim for use as an oral medicament for delivering a controlled substance to a patient so as to obtain a therapeutic, but not a substantial euphoric effect.

31. The composition of any preceding claim for use as a parenterally administered medicament for delivering a controlled substance to a patient so as to obtain a therapeutic, but not a substantial euphoric effect.

32. The composition of any preceding claim for use as a medicament for preventing the euphoric effect of a controlled substance comprising providing a controlled substance covalently bound to a carbohydrate.

## Patentansprüche

1. Pharmazeutische Zusammensetzung, die folgendes umfasst:
eine geregelte Substanz; und
ein Kohlenhydrat, das mit der genannten geregelten Substanz eine kovalente Bindung in einer Art und Weise aufweist, welche die genannte geregelte Substanz pharmakologisch inaktiv macht oder ihre Aktivität deutlich verringert;
wobei die geregelte Substanz aus der Gruppe ausgewählt wird, die folgendes umfasst: Codein, Fentanyl, Hydrocodon, Hydromorphon, Methadon, Morphin, Oxycodon, Propoxyphen, Sufentanyl, Amphetamin und Methylphenidat; und
wobei das Kohlenhydrat weniger als zehn einzelne Kohlenhydrate umfasst.

2. Zusammensetzung nach Anspruch 1, wobei es sich bei dem genannten Kohlenhydrat um Zucker handelt.

3. Zusammensetzung nach Anspruch 2, wobei der genannte Zucker ausgewählt wird aus Ribofuranose, Ribose, Galactose, Galactopyranose, Xylose, Mannofuranose oder Kombinationen dieser.

4. Zusammensetzung nach einem der vorstehenden Ansprüche, wobei das Kohlenhydrat weniger als fünf einzelne Kohlenhydrate umfasst.

5. Zusammensetzung nach einem der vorstehenden Ansprüche, wobei das Kohlenhydrat vier einzelne Kohlenhydrate umfasst.

6. Zusammensetzung nach einem der vorstehenden Ansprüche, wobei das Kohlenhydrat drei einzelne Kohlenhydrate umfasst.

7. Zusammensetzung nach einem der vorstehenden Ansprüche, wobei das Kohlenhydrat zwei einzelne Kohlenhydrate umfasst.

8. Zusammensetzung nach einem der vorstehenden Ansprüche, wobei das Kohlenhydrat ein einziges Kohlenhydrat umfasst.

9. Zusammensetzung nach einem der vorstehenden Ansprüche, wobei es sich bei dem genannten Kohlenhydrat um Ribofuranose handelt.

10. Zusammensetzung nach einem der vorstehenden Ansprüche, wobei das genannte Kohlenhydrat durch eine Ester- oder Karbonatbindung mit der genannten geregelten Substanz gebunden ist.

11. Zusammensetzung nach einem der vorstehenden Ansprüche, wobei es sich bei der genannten geregelten Substanz um Codein handelt.

12. Zusammensetzung nach einem der Ansprüche 1 bis 10, wobei es sich bei dem genannten Analgetikum um Fentanyl handelt.

13. Zusammensetzung nach einem der Ansprüche 1 bis 10, wobei es sich bei dem genannten Analgetikum um Hydrocodon handelt.

14. Zusammensetzung nach einem der Ansprüche 1 bis 10, wobei es sich bei dem genannten Analgetikum um Hydromorphon handelt.

15. Zusammensetzung nach einem der Ansprüche 1 bis 10, wobei es sich bei dem genannten Analgetikum um Levorphanol handelt.

16. Zusammensetzung nach einem der Ansprüche 1 bis 10, wobei es sich bei dem genannten Analgetikum um Methadon handelt.

17. Zusammensetzung nach einem der Ansprüche 1 bis 10, wobei es sich bei dem genannten Analgetikum um Morphin handelt.

18. Zusammensetzung nach einem der Ansprüche 1 bis 10, wobei es sich bei dem genannten Analgetikum um Oxycodon handelt.

19. Zusammensetzung nach einem der Ansprüche 1 bis 10, wobei es sich bei dem genannten Analgetikum um Propoxphen handelt.

20. Zusammensetzung nach einem der Ansprüche 1 bis 10, wobei es sich bei dem genannten Analgetikum um Sufentanyl handelt.

21. Zusammensetzung nach einem der Ansprüche 1 bis 10, wobei es sich bei der genannten geregelten Substanz um Amphetamin handelt.

22. Zusammensetzung nach einem der Ansprüche 1 bis 10, wobei es sich bei der genannten geregelten Substanz um Methylphenidat handelt.

23. Zusammensetzung nach einem der vorstehenden Ansprüche, die widerstandsfähig ist in Bezug auf die Freisetzung der genannten geregelten Substanz in einer pharmakologisch aktiven Form bei einer Verabreichung durch parenterale Injektion, wodurch eine euphorisierende Wirkung verringert oder verhindert wird.

24. Zusammensetzung nach einem der Ansprüche 1 bis 22, welche die genannte geregelte Substanz in einer pharmakologisch aktiven Form freisetzt, wenn sie über einen oralen Weg einem menschlichen Patienten verabreicht wird.

25. Zusammensetzung nach einem der Ansprüche 1 bis 22, die widerstandsfähig ist in Bezug auf die Freisetzung und/oder Absorption der genannten geregelten Substanz in einer pharmakologisch aktiven Form, wenn sie über einen intranasalen Weg verabreicht wird, wodurch eine euphorisierende Wirkung verringert oder verhindert wird.

26. Zusammensetzung nach einem der Ansprüche 1 bis 22, die widerstandsfähig ist in Bezug auf die Freisetzung und/oder Absorption der genannten geregelten Substanz in einer pharmakologisch aktiven Form, wenn sie durch Inhalation verabreicht wird, wodurch eine euphorisierende Wirkung verringert oder verhindert wird.

27. Zusammensetzung nach einem der Ansprüche 1 bis 22, wobei die genannte geregelte Substanz in aktiver Form in Gegenwart von Säure im Magen oder in Gegenwart von Enzymen im Darmtrakt oder im Blutserum freigesetzt wird.

28. Zusammensetzung nach einem der Ansprüche 1 bis 22 mit einer Formulierung für eine orale Verabreichung, wobei die genannte geregelte Substanz in aktiver Form in Gegenwart von Säure im Magen oder in Gegenwart von Enzymen im Darmtrakt freigesetzt wird.

29. Zusammensetzung nach einem der Ansprüche 1 bis 22, die widerstandsfähig ist in Bezug auf die Absorption über die Blut-Gehirn-Schranke der genannten geregelten Substanz in einer pharmakologisch aktiven Form, wenn sie durch parenterale Injektion verabreicht wird, wodurch eine euphorisierende Wirkung verringert oder verhindert wird.

30. Zusammensetzung nach einem der vorstehenden Ansprüche zur Verwendung als ein orales Arzneimittel zur Verabreichung einer geregelten Substanz an einen Patienten, so dass eine therapeutische Wirkung, jedoch keine substanzielle euphorisierende Wirkung erzielt wird.

31. Zusammensetzung nach einem der vorstehenden Ansprüche zur Verwendung als ein parenteral verabreichtes Arzneimittel für die Verabreichung einer geregelten Substanz an einen Patienten, so dass eine therapeutische Wirkung, jedoch keine substanzielle euphorisierende Wirkung erzielt wird.

32. Zusammensetzung nach einem der vorstehenden Ansprüche zur Verwendung als ein Arzneimittel zum Verhindern der euphorisierenden Wirkung einer geregelten Substanz, das Bereitstellen einer geregelten Substanz, die kovalent mit einem Kohlenhydrat gebunden ist, umfassend.

## Revendications

1. Composition pharmaceutique comprenant :
une substance contrôlée ; et
un glucide lié par covalence à ladite substance contrôlée d'une manière qui rend ladite substance contrôlée pharmacologiquement inactive ou diminue sensiblement son activité,
dans laquelle la substance contrôlée est sélectionnée parmi le groupe comprenant : codéine, fentanyle, hydrocodone, hydromorphone, lévorphanol, méthadone, morphine, oxycodone, propoxyphène, sufentanyle, amphétamine et méthylphénidate, et
dans laquelle le glucide comprend moins de 10 glucides individuels.

2. Composition selon la revendication 1, dans laquelle ledit glucide est un sucre.

3. Composition selon la revendication 2, dans laquelle ledit sucre est sélectionné parmi le ribofuranose, le ribose, le galactose, le galactopyranose, le xylose, le mannofuranose ou des combinaisons de ceux-ci.

4. Composition selon l'une quelconque des revendications précédentes, dans laquelle le glucide comprend moins de 5 glucides individuels.

5. Composition selon l'une quelconque des revendications précédentes, dans laquelle le glucide comprend 4 glucides individuels.

6. Composition selon l'une quelconque des revendications précédentes, dans laquelle le glucide comprend 3 glucides individuels.

7. Composition selon l'une quelconque des revendications précédentes, dans laquelle le glucide comprend 2 glucides individuels.

8. Composition selon l'une quelconque des revendications précédentes, dans laquelle le glucide comprend un glucide unique.

9. Composition selon l'une quelconque des revendications précédentes, dans laquelle ledit glucide est le ribofuranose.

10. Composition selon l'une quelconque des revendications précédentes, dans laquelle ledit glucide est lié à ladite substance contrôlée par une liaison ester ou carbonate.

11. Composition selon l'une quelconque des revendications précédentes, dans laquelle ladite substance contrôlée est la codéine.

12. Composition selon les revendications 1 à 10, dans laquelle ledit analgésique est le fentanyle.

13. Composition selon les revendications 1 à 10, dans laquelle ledit analgésique est l'hydrocodone.

14. Composition selon les revendications 1 à 10, dans laquelle ledit analgésique est l'hydromorphone.

15. Composition selon les revendications 1 à 10, dans laquelle ledit analgésique est le lévorphanol.

16. Composition selon les revendications 1 à 10, dans laquelle ledit analgésique est la méthadone.

17. Composition selon les revendications 1 à 10, dans laquelle ledit analgésique est la morphine.

18. Composition selon les revendications 1 à 10, dans laquelle ledit analgésique est l'oxycodone.

19. Composition selon les revendications 1 à 10, dans laquelle ledit analgésique est le propoxyphène.

20. Composition selon les revendications 1 à 10, dans laquelle ledit analgésique est le sufentanyle.

21. Composition selon les revendications 1 à 10, dans laquelle ladite substance contrôlée est une amphétamine.

22. Composition selon les revendications 1 à 10, dans laquelle ladite substance contrôlée est le méthylphénidate.

23. Composition selon l'une quelconque des revendications précédentes, qui est résistante à la libération de ladite substance contrôlée sous une forme pharmacologiquement active lors de l'administration par injection parentérale, diminuant ou empêchant ainsi un effet euphorique.

24. Composition selon les revendications 1 à 22, qui libère ladite substance contrôlée sous une forme pharmacologiquement active lors de l'administration à un patient humain par la voie orale.

25. Composition selon les revendications 1 à 22, qui est résistante à la libération et/ou l'absorption de ladite substance contrôlée sous une forme pharmacologiquement active lors de l'administration par la voie intranasale, diminuant ou empêchant ainsi un effet euphorique.

26. Composition selon les revendications 1 à 22, qui est résistante à la libération et/ou l'absorption de ladite substance contrôlée sous une forme pharmacologiquement active lors de l'administration par inhalation, diminuant ou empêchant ainsi un effet euphorique.

27. Composition selon les revendications 1 à 22, dans laquelle ladite substance contrôlée est libérée sous forme active en présence d'acide dans l'estomac ou en présence d'enzymes dans le tractus intestinal ou le sérum sanguin.

28. Composition selon les revendications 1 à 22, formulée pour l'administration orale, dans laquelle ladite substance contrôlée est libérée sous forme active en présence d'acide présent dans l'estomac ou en présence d'enzymes présentes dans le tractus intestinal.

29. Composition selon les revendications 1 à 22, qui est résistante à l'absorption en travers de la barrière hématoencéphalique de ladite substance contrôlée sous une forme pharmacologiquement active lors de l'administration par injection parentérale, diminuant ou empêchant ainsi un effet euphorique.

30. Composition selon l'une quelconque des revendications précédentes destinée à être utilisée en tant que médicament oral pour fournir une substance contrôlée à un patient de manière à obtenir un effet thérapeutique, mais pas un effet euphorique important.

31. Composition selon l'une quelconque des revendications précédentes destinée à être utilisée en tant que médicament administré par voie parentérale pour fournir une substance contrôlée à un patient de manière à obtenir un effet thérapeutique, mais pas un effet euphorique important.

32. Composition selon l'une quelconque des revendications précédentes destinée à être utilisée en tant que médicament pour empêcher l'effet euphorique d'une substance contrôlée comprenant la fourniture d'une substance contrôlée liée par covalence à un glucide.
